# EUROPÄISCHE PATENTSCHRIFT

(11) **EP 2 134 369 B1**
(45) Veröffentlichungstag und Bekanntmachung des Hinweises auf die Patenterteilung: **02.01.2019**
(21) Anmeldenummer: 08735042.7
(22) Anmeldetag: 04.04.2008
(51) Int. Cl.: A61K 47/00, A61K 36/185

(54) **VERFAHREN ZUR HERSTELLUNG VON TROCKENEXTRAKTEN AUS PELARGONIUM SIDOIDES UND PELARGONIUM RENIFORME**
METHOD FOR PRODUCING DRY EXTRACTS OF PELARGONIUM SIDOIDES AND PELARGONIUM RENIFORME
PROCÉDÉ DE FABRICATION D'EXTRAITS SECS DE PELARGONIUM SIDOIDES ET PELARGONIUM RENIFORME

(30) Priorität: 17.04.2007 DE 102007018079
(43) Veröffentlichungstag der Anmeldung: 23.12.2009
(73) Patentinhaber: Dr. Willmar Schwabe GmbH & Co. KG, 76227 Karlsruhe (DE)
(72) Erfinder: HERRMANN, Joachim, 68789 St. Leon-Rot (DE); THÖLE, Marc, 76227 Karlsruhe (DE)
(74) Vertreter: Adam, Holger
(86) Internationale Anmeldenummer: PCT/EP2008/002720
(87) Internationale Veröffentlichungsnummer: WO 2008/125239

(56) Entgegenhaltungen:
- WO-A-92/21357
- WO-A-99/32130
- WO-A-2007/009446

## Beschreibung

Die Erfindung betrifft Herstellungsverfahren zur Gewinnung von Trockenextrakten aus Pelargonium sidoides und/oder Pelargonium reniforme, Trockenextrakte die nach diesen Verfahren erhältlich sind, sowie Arzneimittel und Lebensmittel, die solche Extrakte enthalten.
Aus den im südlichen Afrika beheimateten Pelargonium-Arten Pelargonium sidoides und/oder Pelargonium reniforme gewonnene Zubereitungen werden dort traditionell zur therapeutischen Behandlung von Atemwegserkrankungen und gastrointestinalen Beschwerden angewendet.
Die Wirksamkeit eines wässrig-ethanolischen Flüssigextrakts aus den Wurzeln von Pelargonium sidoides, EPs 7630, bei Infektionen der Atemwege und des HNO-Bereichs konnte inzwischen in zahlreichen klinischen Studien und Anwendungsbeobachtungen belegt werden (Kolodziej et al., Deutsche Apotheker Zeitung 143 (12): 55-64 (2003)).
Die Wirkung des Extrakts wird durch mehrere therapeutisch wirksame Komponenten hervorgerufen. Als wichtige therapeutische Komponenten in Pelargonium sidoides gelten Gerbstoffe und Cumarin-Derivate. Derartige Komponenten sind gleichfalls in Extrakten aus Pelargonium reniforme enthalten.
Das Europäische Arzneibuch teilt Extrakte nach ihrer Konsistenz ein in flüssige (Flüssigextrakte und Tinkturen), halbfeste (Dickextrakte) und feste (Trockenextrakte) Zubereitungen. Trockenextrakte werden durch Verdunstung bzw. Abziehen des zur Herstellung verwendeten Lösungsmittels hergestellt und weisen üblicherweise einen Trocknungsverlust oder Wassergehalt von maximal 5 Gewichtsprozent auf. Sie haben gegenüber flüssigen und halbfesten Extrakten viele Vorteile. Sie weisen eine bessere Stabilität auf, sind leichter handhabbar und können zur Herstellung von festen galenischen Darreichungsformen verwendet werden. Die direkte Verwendung eines wässrig-ethanolischen Flüssigextrakts ist insbesondere in den Fällen ausgeschlossen, in denen eine alkoholfreie flüssige Darreichungsform gewünscht wird, z.B. zur Anwendung bei Kindern.

Pflanzliche Trockenextrakte sind z. B. aus der EP0589921B1 und der EP1037647B2 bekannt. Diese Trockenextrakte enthalten u.a. Trägerstoffe.

Die EP0589921B1 betrifft pflanzliche Dick- und/oder Trockenextrakte mit gleichem oder annähernd gleichem Wirkstoffspektrum eines entsprechenden Flüssigextraktes, deren Verwendung sowie ein Verfahren zu ihrer Herstellung. Der EP0589921B1 liegt das Problem zugrunde, dass flüchtige Drogenbestandteile von Flüssigextrakten bei herkömmlicher Trocknung durch Verdunstung des Lösungsmittels in den resultierenden Dick- und/oder Trockenextrakten nicht mehr vollständig enthalten sind. Die offenbarten Extrakte können weiterhin pharmazeutische Hilfsstoffe, Trägerstoffe und/oder Sprengmittel enthalten. Als bevorzugte Stoffe sind unter anderem Einfach- und/oder Mehrfachzucker sowie Cellulose, Cellulosederivate, Stärke und Stärkederivate genannt. Durch die Zugabe der Hilfsstoffe, die nach dem Entfernen des Lösungsmittels der ursprünglichen Flüssigextrakte erfolgt, soll verhindert werden, dass die flüchtigen Bestandteile bei der späteren Weiterverarbeitung zu Pharmazeutika in relevantem Ausmaß entweichen.

Die EP1037647B2 betrifft peroral applizierbare Arzneipflanzentrockenextrakte aus Passiflora, Agnus castus, Crataegus, Ginkgo, Brennesselextrakt, Baldrian, Cimicifuga-Wurzel oder Wurzelstock und/oder Cynara, wobei die nicht flüchtige Phase des Extraktes an einen bei Raumtemperatur festen Träger I, ausgewählt aus Polyethylenglykolen, Polyvinylalkoholen, Polyvidonacetat und/oder Polyvinylpyrrolidon, und weiterhin einen Träger II, ausgewählt aus alkoholunlöslichen, wasserunlöslichen, in Wasser quellbaren, bei Raumtemperatur festen Trägern oder Erdalkalimetall- und/oder Alkalimetallcarbonaten einschließlich Hydrogencarbonaten in mikrodisperser Form und/oder in Form einer halbfesten oder festen Lösung gebunden ist, gegebenenfalls neben weiteren Hilfs- und/oder Zusatzstoffen. Solche Extrakte zeichnen sich durch eine in Ausmaß und Geschwindigkeit definierte Freisetzung der Pflanzeninhaltsstoffe aus.

WO 2007/009446 offenbart ein Verfahren zur Herstellung von ethanolfreien Extrakten des Wurzelmaterials von Pelargonium sidoides, Pelargonium reniforme sowie anderer Pflanzen der Gattung Pelargonium, wobei als Lösungs- und Extraktionsmedium z.B. Wasser und ein einwertiger Alkohol mit mindestens drei Kohlenstoffatomen (z.B. Propanol) oder Wasser und zumindest ein mehrwertiger Alkohol, wie z.B. Zucker, Zuckeralkohole, Sorbitol oder Xylitol verwendet wird.

Bei der Herstellung von Pelargonium-Trockenextrakten besteht jedoch das Problem, dass sich durch direkte Trocknung von Pelargonium-Flüssigextrakten erhaltene Trockenextrakte auch in einem großen Lösungsmittelüberschuss in physiologisch verträglichen, vorwiegend wässrigen oder wässrig-alkoholischen Lösungsmitteln einschließlich Gemischen aus Wasser und Polyolen und ggf. Alkoholen, nur unvollständig lösen (vgl. Vergleichsbeispiele 1 - 2). Dadurch wird zum einen die Herstellung von flüssigen Zubereitungen aus diesen Trockenextrakten erschwert, zum anderen kann so generell die Wirksamkeit der Trockenextrakte beeinträchtigt sein.
Aufgabe der vorliegenden Erfindung ist daher die Bereitstellung von Trockenextrakten aus Pelargonium sidoides und/oder reniforme mit verbesserter Löslichkeit.

Die Aufgabe wird gelöst durch ein Verfahren zur Herstellung eines Trockenextrakts aus Pelargonium sidoides und/oder Pelargonium reniforme mit verbesserter Löslichkeit in physiologisch verträglichen, vorwiegend wässrigen oder wässrig-alkoholischen Lösungsmitteln einschließlich Gemischen aus Wasser und Polyolen und ggf. Alkoholen, gekennzeichnet durch folgende Verfahrensschritte:
(a) Herstellung einer wässrigen oder wässrig-alkoholischen oder wässrig-ketonischen Lösung eines Ausgangsextrakts aus Pelargonium sidoides und/oder Pelargonium reniforme, wobei es sich bei dem Alkohol in der wässrig-alkoholischen Lösung um einen einwertigen C₁-C₃-Alkohol, ausgewählt aus Methanol, Ethanol, 1-Propanol und 2-Propanol handelt,
(b) Zugabe eines festen Trägerstoffs oder mehrerer fester Trägerstoffe, unabhängig voneinander ausgewählt aus der Gruppe bestehend aus Fructose, Galactose, Glucose, Xylose, alpha-Cyclodextrin, beta-Cyclodextrin, gamma-Cyclodextrin, Hydroxypropylbetadex, Lactose, Lactulose, Maltose, Raffinose, Saccharose, Trehalose, Chitosan, Chitosanhydrochlorid, Dextran, Dextrin, Guargalactomannan, Arabisches Gummi, Hydroxyethylcellulose, Hydroxypropylcellulose, Hydroxypropylmethylcellulose, Inulin, Maltodextrin, Methylcellulose, Methylhydroxyethylcellulose, Polydextrose, Erythritol, Isomalt, Lactitol, Maltitol, Mannitol, Sorbitol und Xylitol, wobei das Massenverhältnis von Trägerstoff zum Trockenrückstand der Lösung des Ausgangsextrakts 1:4 bis 9:1 beträgt, und
(c) Einengen und Trocknen der so erhaltenen Extraktlösung zum Trockenextrakt.

Nach dem erfindungsgemäßen Verfahren hergestellte Trockenextrakte sind in physiologisch verträglichen Lösungsmitteln mindestens wenig löslich, d.h. sie lösen sich nach dem "Europäischen Arzneibuch", Ausgabe 5, in einem Verhältnis von mindestens 1 Gramm Trockenextrakt zu 100 Millilitern Lösungsmittel, praktisch restlos auf und ergeben dementsprechend eine klare oder opaleszierende Lösung ohne Bodensatz, wobei die Opaleszenz nicht stärker ist als die der Opaleszenz-Referenzsuspension des "Europäischen Arzneibuchs", Ausgabe 5 (entsprechend 60 NTU = Nephelometric Turbidity Units).

Überraschend wurde gefunden, dass die Löslichkeit von Trockenextrakten aus Pelargonium sidoides und/oder reniforme signifikant verbessert wird, wenn den zur Herstellung verwendeten Extraktlösungen vor der Überführung in eine feste Form durch Trocknung Trägerstoffe, die aus der Gruppe der Saccharide und Zuckeralkohole ausgewählt sind, zugesetzt werden. Dieser Effekt ist besonders überraschend, da die Lösungseigenschaften von konventionell hergestellten Trockenextrakten in physiologisch verträglichen Lösungsmitteln durch einfaches Zumischen dieser Trägerstoffe nicht verbessert werden (vgl. Vergleichsbeispiele 3 - 8).

Die verbesserte Löslichkeit der erfindungsgemäßen Trockenextrakte ist besonders vorteilhaft, wenn die Trockenextrakte zusammen mit den üblichen Hilfsstoffen zu (überzogenen) Tabletten verarbeitet werden. In diesem Fall kann durch die Verwendung des erfindungsgemäßen Trockenextrakts eine besonders gute Wirkstofffreisetzung erreicht werden. Diese wird typischerweise gemäß der Methode 2.9.3.5 des Europäischen Arzneibuchs, Ausgabe 5, "Prüfung der Wirkstofffreisetzung aus festen Arzneiformen" nachgewiesen. Eine gute Wirkstofffreisetzung aus der Darreichungsform ist Voraussetzung für eine gute Wirksamkeit.

Die für das Verfahren zur Herstellung der erfindungsgemäßen Trockenextrakte aus Pelargonium sidoides und/oder reniforme einzusetzenden Extraktlösungen (d.h. Lösungen des Ausgangsextrakts) können beispielsweise gewonnen werden, indem zunächst getrocknete und zerkleinerte Wurzeln von Pelargonium sidoides und/oder Pelargonium reniforme mit Wasser oder einem oder mehreren wässrig-alkoholischen Lösungsmitteln oder einem oder mehreren wässrig-ketonischen (z.B. wässrig-acetonischen) Lösungsmitteln in herkömmlicher Weise, beispielsweise bei Temperaturen von 10 bis 100 °C, extrahiert werden. Der Drogenrückstand wird gegebenenfalls leicht ausgepresst und der rohe Extrakt wird gegebenenfalls filtriert. Bevorzugt werden zur Herstellung der Lösung des Ausgangsextrakts Gemische aus Wasser und einem einwertigen C₁-C₃-Alkohol, ausgewählt aus Methanol, Ethanol, 1-Propanol und 2-Propanol verwendet.

Der Wasseranteil der wässrig-alkoholischen bzw. wässrig-ketonischen Lösungsmittel beträgt vorzugsweise mindestens 50 Gew.-% und bevorzugt höchstens 95 Gew.-%. Bevorzugt erfolgt die Herstellung des Flüssigextrakts durch Perkolation mit einem wässrig-ethanolischen Lösungsmittel, gegebenenfalls nach einem zuvor erfolgten Anmaischen mit einem wässrig-ethanolischen Lösungsmittel entsprechend der EP 1 429 795.
Weitere geeignete Extraktlösungen sind beispielsweise auch in der DE 102004063910, insbesondere im Absatz [0017] und in den Beispielen 3 und 4 beschrieben. Der Offenbarungsgehalt der beiden letztgenannten Druckschriften ist bezüglich der Herstellung der Extraktlösungen ausdrücklich unter Bezugnahme hierin umfasst.
Anschließend wird ein fester Trägerstoff in dem so erhaltenen Flüssigextrakt gelöst. Alternativ können auch mehrere feste Trägerstoffe eingesetzt werden. Das Massenverhältnis von Trägerstoff bzw. Trägerstoffen zu Trockenrückstand (bestimmt nach dem "Europäischen Arzneibuch", Ausgabe 5, durch 3 h Trocknen bei 100 - 105 °C) der Extraktlösung beträgt 1:4 bis 9:1, bevorzugt 1:1 bis 6:1, insbesondere 2:1 bis 5:1. Die Lösung wird unter Anwendung üblicher Verfahren, beispielsweise bei einem Druck von 0,001 bar bis Atmosphärendruck und einer Temperatur von 20 bis 100 °C, eingeengt und getrocknet. Der Zusatz des Trägerstoffs bzw. der Trägerstoffe kann alternativ auch während der Einengung erfolgen.

Geeignete Trägerstoffe sind die Monosaccharide Fructose, Galactose, Glucose, Xylose und/oder die Oligosaccharide α-Cyclodextrin, β-Cyclodextrin, γ-Cyclodextrin, Hydroxypropylbetadex, Lactose, Lactulose, Maltose, Raffinose, Saccharose, Trehalose und/oder die Polysaccharide Chitosan, Chitosanhydrochlorid, Dextran, Dextrin, Guargalactomannan, Arabisches Gummi, Hydroxyethylcellulose, Hydroxypropylcellulose, Hydroxypropylmethylcellulose, Inulin, Maltodextrin, Methylcellulose, Methylhydroxyethylcellulose, Polydextrose,und/oder die Zuckeralkohole Erythritol, Isomalt, Lactitol, Maltitol, Mannitol, Sorbitol, Xylitol.
Gegenstand der Erfindung sind auch die nach dem erfindungsgemäßen Verfahren erhältlichen Trockenextrakte aus Pelargonium sidoides und/oder reniforme, sowie Arzneimittel und Lebensmittel, welche diese Trockenextrakte und weitere, für Arzneimittel bzw. Lebensmittel zulässige Bestandteile enthalten.

Unter Lebensmitteln sind hierbei insbesondere diätetische Lebensmittel, Nahrungsergänzungsmittel, sowie "medical food", "health food" und "dietary supplements" zu verstehen.

Darüber hinaus werden Zubereitungen, beschrieben die diese Trockenextrakte, ggf. in Kombination mit anderen Stoffen, wie z.B. Wirk- und/oder Hilfsstoffen, enthalten.

Diese Zubereitungen können beispielsweise Medizinprodukte, Kosmetika oder Bedarfsgegenstände sein. Die erfindungsgemäßen Trockenextrakte können zusammen mit den üblichen Hilfsstoffen zu festen Zubereitungen wie Pulvern, Granulaten, Pellets, Tabletten, Kapseln oder überzogenen Tabletten verarbeitet werden. Als Hilfsstoffe können übliche Füll-, Binde-, Spreng-, Schmiermittel sowie gegebenenfalls Geruchs- und Geschmacksstoffe und Überzugsmittel für überzogene Tabletten verwendet werden. Als Hilfsstoffe zur Herstellung von Weichkapseln können übliche Öle und Fette als Füllmassen verwendet werden, die Weichkapselhülle kann z.B. aus Gelatine bestehen. Die erfindungsgemäßen Trockenextrakte können zusammen mit üblichen Hilfsstoffen zu flüssigen Zubereitungen wie Lösungen, Sprays, Emulsionen und Suspensionen verarbeitet werden. Als Hilfsstoffe können übliche Lösungsmittel, Lösungsvermittler, Stabilisatoren sowie Geruchs- und Geschmacksstoffe verwendet werden. Die Dosierung erfolgt dabei so, dass pro Tag eine Trockenextraktmenge zugeführt wird, die 2 bis 1000 mg, bevorzugt 5 bis 400 mg und besonders bevorzugt 10 bis 200 mg Trockenrückstand des zur Herstellung verwendeten Flüssigextrakts entspricht.

### Beispiele

In den Vergleichsbeispielen 1 bis 8 und den Beispielen 9 bis 14 wurden folgende Lösungsmittel A und B verwendet:

**Lösungsmittel A:**

| | |
|---|---|
| Ethanol 96 Vol. % | 10 Massenteile |
| Glycerol 85 Gew. % | 20 Massenteile |
| Wasser | 70 Massenteile |

**Lösungsmittel B:**

| | |
|---|---|
| Glycerol 85 Gew. % | 10 Massenteile |
| Xylitol | 10 Massenteile |
| Wasser | 80 Massenteile |

### Vergleichsbeispiele 1 - 8:

14 kg gemahlene Wurzel von Pelargonium sidoides wurden mit 28 kg Ethanol (35 Gew. %) versetzt und 20 h bei Raumtemperatur gelagert. Danach wurde die Mischung für 10 h mit 112 kg Ethanol (6 Gew. %) perkoliert und anschließend filtriert. Der Trockenrückstand des Filtrats betrug 1,78 Gew. %.

50 kg dieses Flüssigextrakts wurden bei 50 °C unter Vakuum (bis 18 mbar) getrocknet.

Je 1 g des erhaltenen Trockenextraktes wurden, gegebenenfalls nach intensivem Mischen mit 4,55 g eines Trägerstoffs in einer Reibschale, mit 100 ml Lösungsmittel A oder B gemischt.

| Vergleichsbeispiel Nr. | 1 | 2 | 3 | 4 | 5 | 6 | 7 | 8 |
|---|---|---|---|---|---|---|---|---|
| Trockenextrakt | 1,00 g | 1,00 g | 1,00 g | 1,00 g | 1,00 g | 1,00 g | 1,00 g | 1,00 g |
| Mannitol | --- | --- | 4,55 g | 4,55 g | --- | --- | --- | --- |
| Saccharose | --- | --- | --- | --- | 4,55 g | 4,55 g | --- | --- |
| Maltodextrin | --- | --- | --- | --- | --- | --- | 4,55 g | 4,55 g |
| Opaleszenz Überstand [NTU] | 1,5 | 6,5 | 1,84 | 3,8 | 1,8 | 4,2 | 14 | 115 |
| Lösungsmittel | A | B | A | B | A | B | A | B |
| Bodensatz | + | + | + | + | + | + | + | + |

Der Trockenextrakt war nicht vollständig löslich. Alle Lösungen wiesen einen Bodensatz auf.

### Beispiele 9 -10 (erfindungsgemäße Beispiele):

14 kg gemahlene Wurzel von Pelargonium sidoides wurden mit 28 kg Ethanol (35 Gew. %) versetzt und 20 h bei Raumtemperatur gelagert. Danach wurde die Mischung für 10 h mit 112 kg Ethanol (6 Gew. %) perkoliert und anschließend filtriert. Der Trockenrückstand des Filtrats betrug 1,78 Gew. %.

In 15,4 kg dieses Flüssigextrakts wurden 1,25 kg Mannitol gelöst. Die Lösung wurde bei 50 °C unter Vakuum (bis 18 mbar) getrocknet.

Je 5,55 g des erhaltenen Trockenextrakts (entsprechend 1 g Nativanteil und 4,55 g Mannitol) wurden mit 100 ml Lösungsmittel A oder B gemischt.

| Beispiel Nr. | 9 | 10 |
|---|---|---|
| Trockenextrakt mit Mannitol | 5,55 g | 5,55 g |
| Opaleszenz Lösung [NTU] | 3,2 | 2,6 |
| Lösungsmittel | A | B |
| Bodensatz | - | - |

Der Trockenextrakt löste sich vollständig. Beide Lösungen wiesen keinen Bodensatz auf.

### Beispiele 11 -12 (erfindungsgemäße Beispiele):

14 kg gemahlene Wurzel von Pelargonium sidoides wurden mit 28 kg Ethanol (35 Gew. %) versetzt und 20 h bei Raumtemperatur gelagert. Danach wurde die Mischung für 10 h mit 112 kg Ethanol (6 Gew. %) perkoliert und anschließend filtriert. Der Trockenrückstand des Filtrats betrug 1,78 Gew. %.

In 14,7 kg dieses Flüssigextrakts wurden 1,19 kg Saccharose gelöst. Die Lösung wurde bei 50 °C unter Vakuum (bis 18 mbar) getrocknet.

Je 5,55 g des erhaltenen Trockenextrakts (entsprechend 1 g Nativanteil und 4,55 g Saccharose) wurden mit 100 ml Lösungsmittel A oder B gemischt.

| Beispiel Nr. | 11 | 12 |
|---|---|---|
| Trockenextrakt mit Saccharose | 5,55 g | 5,55 g |
| Opaleszenz Lösung [NTU] | 4,2 | 2,0 |
| Lösungsmittel | A | B |
| Bodensatz | - | - |

Der Trockenextrakt löste sich vollständig. Beide Lösungen wiesen keinen Bodensatz auf.

### Beispiele 13 - 14 (erfindungsgemäße Beispiele):

14 kg gemahlene Wurzel von Pelargonium sidoides wurden mit 28 kg Ethanol (35 Gew. %) versetzt und 20 h bei Raumtemperatur gelagert. Danach wurde die Mischung für 10 h mit 112 kg Ethanol (6 Gew. %) perkoliert und anschließend filtriert. Der Trockenrückstand des Filtrats betrug 1,78 Gew. %.

In 16,5 kg dieses Flüssigextrakts wurden 1,34 kg Maltodextrin gelöst. Die Lösung wurde bei 50 °C unter Vakuum (bis 18 mbar) getrocknet.

Je 5,55 g des erhaltenen Trockenextrakts (entsprechend 1 g Nativanteil und 4,55 g Maltodextrin) wurden mit 100 ml Lösungsmittel A oder B gemischt.

| Beispiel Nr. | 13 | 14 |
|---|---|---|
| Trockenextrakt mit Maltodextrin | 5,55 g | 5,55 g |
| Opaleszenz Lösung [NTU] | 4,7 | 33 |
| Lösungsmittel | A | B |
| Bodensatz | - | - |

Der Trockenextrakt löste sich vollständig. Beide Lösungen wiesen keinen Bodensatz auf.

## Patentansprüche

1. Verfahren zur Herstellung eines Trockenextrakts aus Pelargonium sidoides und/oder Pelargonium reniforme mit verbesserter Löslichkeit in physiologisch verträglichen, vorwiegend wässrigen oder wässrig-alkoholischen Lösungsmitteln einschließlich Gemischen aus Wasser und Polyolen und ggf. Alkoholen, **gekennzeichnet durch** folgende Verfahrensschritte:
(a) Herstellung einer wässrigen oder wässrig-alkoholischen oder wässrig-ketonischen Lösung eines Ausgangsextrakts aus Pelargonium sidoides und/oder Pelargonium reniforme, wobei es sich bei dem Alkohol in der wässrig-alkoholischen Lösung um einen einwertigen C₁-C₃-Alkohol, ausgewählt aus Methanol, Ethanol, 1-Propanol und 2-Propanol handelt,
(b) Zugabe eines festen Trägerstoffs oder mehrerer fester Trägerstoffe, unabhängig voneinander ausgewählt aus der Gruppe bestehend aus Fructose, Galactose, Glucose, Xylose, α-Cydodextrin, β-Cyclodextrin, γ-Cyclodextrin, Hydroxypropylbetadex, Lactose, Lactulose, Maltose, Raffinose, Saccharose, Trehalose, Chitosan, Chitosanhydrochlorid, Dextran, Dextrin, Guargalactomannan, Arabisches Gummi, Hydroxyethylcellulose, Hydroxyprpylcellulose Hydroxypropylmethylcellulose, Inulin, Maltodextrin, Methylcellulose Methylhydroxyethylcellulose, Polydextrose, Erythritol, Isomalt, Lactitol, Maltitol, Mannitol, Sorbitol und Xylitol, wobei das Massenverhältnis von Trägerstoff zum Trockenrückstand der Lösung des Ausgangsextrakts 1:4 bis 9:1 beträgt, und
(c) Einengen und Trocknen der so erhaltenen Extraktlösung zum Trockenextrakt.

2. Verfahren nach Anspruch 1, bei dem zur Herstellung der Lösung des Ausgangsextrakts Wasser-Methanol-Gemische, Wasser-Ethanol-Gemische, Wasser-1-Propanol-Gemische, Wasser-2-Propanol-Gemische oder Wasser-Aceton-Gemische verwendet werden.

3. Verfahren nach einem der Ansprüche 1 oder 2, bei dem zur Herstellung der Lösung des Ausgangsextrakts Wasser-Methanol-Gemische, Wasser-Ethanol-Gemische, Wasser-1-Propanol-Gemische, Wasser-2-Propanol-Gemische oder Wasser-Aceton-Gemische verwendet werden, wobei der Wasseranteil der Gemische mindestens 50 Gew.-% beträgt.

4. Verfahren nach einem der Ansprüche 2 oder 3, wobei der Wasseranteil der zur Herstellung der Lösung des Ausgangsextrakts verwendeten Gemische höchstens 95 Gew.-% beträgt.

5. Verfahren nach einem der Ansprüche 1 bis 4, bei dem das Massenverhältnis von Trägerstoff zum Trockenrückstand der Lösung des Ausgangsextrakts 1:1 bis 6:1 beträgt.

6. Verfahren nach einem der Ansprüche 1 bis 4, bei dem das Massenverhältnis von Trägerstoff zum Trockenrückstand der Lösung des Ausgangsextrakts 2:1 bis 5:1 beträgt.

7. Trockenextrakt aus Pelargonium sidoides und/oder Pelargonium reniforme, erhältlich nach einem der Ansprüche 1 bis 6.

8. Arzneimittel, enthaltend einen Trockenextrakt gemäß Anspruch 7 und weitere für Arzneimittel zulässige Bestandteile.

9. Lebensmittel, enthaltend einen Trockenextrakt gemäß Anspruch 7 und weitere für Lebensmittel zulässige Bestandteile.

## Claims

1. Method for preparing a dry extract from *Pelargonium sidoides* and/or *Pelargonium reniforme* with improved solubility in physiologically compatible, primarily aqueous and or aqueous-alcoholic solvents including mixtures of water and polyols and, optionally, alcohols, **characterised by** the following process steps:
(a) preparing an aqueous or aqueous-alcoholic or aqueous-ketonic solution of a starting extract from *Pelargonium sidoides* and/or *Pelargonium reniforme,* the alcohol in the aqueous-alcoholic solution being a monohydric C₁-C₃ alcohol selected from methanol, ethanol, 1-propanol and 2-propanol,
(b) adding a solid carrier substance or several solid carrier substances independently selected from the group consisting of fructose, galactose, glucose, xylose, α-cyclodextrin, β-cyclodextrin, γ-cyclodextrin, hydroxypropyl betadex, lactose, lactulose, maltose, raffinose, saccharose, trehalose, chitosan, chitosan hydrochloride, dextran, dextrin, guargalactomannan, gum arabic, hydroxyethyl cellulose, hydroxypropyl cellulose, hydroxypropylmethyl cellulose, inulin, maltodextrin, methylcellulose, methylhydroxyethyl cellulose, polydextrose, erythritol, isomalt, lactilol, maltitol, mannitol, sorbitol, and xylitol, the mass ratio of the carrier substance to the dry residue of the solution of the starting extract being 1 : 4 to 9 : 1, and
(c) evaporating and drying the extract solution thus obtained to yield the dry extract.

2. Method according to claim 1 wherein water-methanol mixtures, water-ethanol mixtures, water-1-propanol mixtures, water-2-propanol mixtures or water-acetone mixtures are used to prepare the solution of the starting extract.

3. Method according to any of the claims 1 or 2 wherein water-methanol mixtures, water-ethanol mixtures, water-1-propanol mixtures, water-2-propanol mixtures or water-acetone mixtures are used to prepare the solution of the starting extract, the water proportion of the mixtures being at least 50 wt.-%.

4. Method according to any of the claims 2 or 3, wherein the water proportion of the mixture used for preparing the solution of the starting extract is 95 wt.-% maximum.

5. Method according to any of the claims 1 to 4, wherein the mass ratio of the carrier substance to the dry residue of the solution of the starting extract is 1 : 1 to 6 : 1.

6. Method according to any of the claims 1 to 4 wherein the mass ratio of the carrier substance to the dry residue of the solution of the starting extract is 2 : 1 to 5 : 1.

7. Dry extract from *Pelargonium sidoides* and/or *Pelargonium reniforme* which may be obtained according to any of the claims 1 to 6.

8. Pharmaceutical product containing a dry extract according to claim 7 and other components allowed for pharmaceuticals.

9. Food product containing a dry extract according to claim 7 and other components allowed for food products.

## Revendications

1. Procédé de préparation d'un extrait sec de Pelargonium sidoides et/ou de Pelargonium reniforme qui a une solubilité améliorée dans des solvents physiologiquement acceptables, principalement aqueux ou hydroalcooliques, y compris des mélanges d'eau et de polyols et, le cas échéant, d'alcools, **caractérisé par** les étapes de procédé suivantes:
(a) la préparation d'une solution aqueuse ou hydroalcoolique ou aqueuse cétonique d'un extrait de départ de Pelargonium sidoides et/ou de Pelargonium reniforme, où l'alcool dans la solution hydroalcoolique est un alcool monohydrique en C₁-C₃ choisi parmi le méthanol, l'éthanol, le propanol-1 et le propanol-2,
(b) l'ajout d'un ou plusieurs supports solides choisi(s) indépendamment dans le groupe constitué du fructose, du galactose, du glucose, du xylose, de la cyclodextrine alpha, de la cyclodextrine béta, de la cyclodextrine gamma, de l'hydroxypropylbétadex, du lactose, du lactulose, du maltose, du raffinose, du saccharose, du tréhalose, du chitosane, de l'hydrochlorure de chitosane, du dextrane, de la dextrine, du guar galactomannane, de la gomme arabique, de l'hydroxyéthylcellulose, de l'hydroxypropylcellulose, de l'hydroxypropylméthylcellulose, de l'inuline, de la maltodextrine, de la méthylcellulose, de la méthyl-hydroxyéthylcellulose, du polydextrose, de l'érythritol, de l'isomalt, du lactitol, du maltitol, du mannitol, du sorbitol et du xylitol, dans lequel le rapport en masse du support au résidu sec de la solution de l'extrait de départ est de 1:4 à 9:1, et
(c) la concentration et le séchage de la solution d'extrait ainsi obtenue pour obtenir un extrait sec.

2. Procédé selon la revendication 1, dans lequel des mélanges eau-méthanol, des mélanges eau-éthanol, des mélanges eau-propanol-1, des mélanges eau-propanol-2 ou des mélanges eau-acétone sont utilisés pour préparer la solution de l'extrait de départ.

3. Procédé selon l'une quelconque des revendications 1 ou 2, dans lequel des mélanges eau-méthanol, des mélanges eau-éthanol, des mélanges eau-propanol-1, des mélanges eau-propanol-2 ou des mélanges eau-acétone sont utilisés pour préparer la solution de l'extrait de départ, dans lequel la teneur en eau des mélanges est d'au moins 50% en poids.

4. Procédé selon l'une quelconque des revendications 2 ou 3, dans lequel la teneur en eau des mélanges utilisés pour préparer la solution de l'extrait de départ est d'au plus 95% en poids.

5. Procédé selon l'une quelconque des revendications 1 à 4, dans lequel le rapport en masse du support au résidu sec de la solution de l'extrait de départ est de 1:1 à 6:1.

6. Procédé selon l'une quelconque des revendications 1 à 4, dans lequel le rapport en masse du support au résidu sec de la solution de l'extrait de départ est de 2:1 à 5:1.

7. Extrait sec de Pelargonium sidoides et/ou Pelargonium reniforme, pouvant être obtenu selon l'une des revendications 1 à 6.

8. Médicament contenant un extrait sec selon la revendication 7 et d'autres ingrédients admissible pour des médicaments.

9. Aliment contenant un extrait sec selon la revendication 7 et d'autres composants admissible pour des aliments.
